(19) Europäisches Patentamt
European Patent Office
Office européen des brevets

(11) **EP 4 486 238 B1**

(12) **EUROPEAN PATENT SPECIFICATION**

(45) Date of publication and mention
of the grant of the patent:
**26.11.2025 Bulletin 2025/48**

(21) Application number: **23701474.1**

(22) Date of filing: **20.01.2023**

(51) International Patent Classification (IPC):
***A61B 18/12*** (2006.01)

(52) Cooperative Patent Classification (CPC):
**A61B 18/1233; A61B 18/12; A61B 18/1206;**
A61B 2018/1273; A61B 2018/128

(86) International application number:
**PCT/EP2023/051444**

(87) International publication number:
**WO 2023/165759 (07.09.2023 Gazette 2023/36)**

(54) **ENERGY CONVEYING DEVICE, GENERATOR DEVICE, AND ELECTROSURGICAL APPARATUS FOR REDUCING LEAKAGE A CURRENT WITH AN ENERGY CONVEYING DEVICE**

ENERGIEFÜHRUNGSVORRICHTUNG, GENERATORVORRICHTUNG UND ELEKTROCHIRURGISCHES GERÄT ZUR REDUZIERUNG VON LECKAGEN EINES STROMS MIT EINER ENERGIEFÜHRUNGSVORRICHTUNG

DISPOSITIF DE TRANSPORT D'ÉNERGIE, DISPOSITIF GÉNÉRATEUR ET APPAREIL ÉLECTROCHIRURGICAL DE RÉDUCTION DE FUITE D'UN COURANT AVEC UN DISPOSITIF DE TRANSPORT D'ÉNERGIE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **04.03.2022 GB 202203053**

(43) Date of publication of application:
**08.01.2025 Bulletin 2025/02**

(73) Proprietor: **Creo Medical Limited**
**Chepstow Gwent NP16 5UH (GB)**

(72) Inventor: **WHITE, Malcolm**
**Chepstow Gwent NP16 5UH (GB)**

(74) Representative: **Mewburn Ellis LLP**
**Aurora Building**
**Counterslip**
**Bristol BS1 6BX (GB)**

(56) References cited:
**EP-A2- 1 854 423       US-A- 3 946 738**
**US-A- 4 615 330       US-A1- 2012 147 507**
**US-A1- 2019 274 717**

**Description**

FIELD OF THE INVENTION

The invention relates to an energy conveying device for conveying radiofrequency (RF) electromagnetic energy and/or microwave electromagnetic energy, which comprises a first conductive line and a second conductive line for conveying a radiofrequency electromagnetic energy and/or microwave electromagnetic energy having a first frequency. Further, the invention relates to a generator device for generating and/or conveying radiofrequency electromagnetic energy and/or microwave electromagnetic energy, comprising the energy conveying device. The invention also refers to an electrosurgical apparatus comprising the generator device.

BACKGROUND TO THE INVENTION

The Standard BS EN 60601-2-2:2009 requires that:

- Any patient circuit specifically designed for bipolar application shall be isolated from earth and from other applied parts at both high and low frequencies.
- The high-frequency (hf) leakage current flowing from either pole of the bipolar output to earth and to the neutral electrode via a 200 Ω non-inductive resistor in each line shall not exceed the value which produces a power in a 200 Ω non-inductive resistor equal to 1 % of the maximum bipolar rated output power, with all output controls set to maximum.

The leakage current for each of the two lines of a bipolar feed to ground and neutral is often caused by stray, leakage, or unwanted capacitance between the opposite line and ground.

Isolation of the bipolar feed from ground and neutral is often achieved using an isolation transformer. This isolation transformer can also introduce capacitance. Approximately half of this capacitance can contribute to the leakage capacitance on each line of the bipolar feed.

In one example, at 400 kHz, with a 1 kΩ load, the total leakage capacitance on one line, that would keep the power in the 200 Ω test resistor on the opposite line below the set limit, would be about 80 pF.

The isolating transformer and associated circuitry may have a capacitance of 60 pF. This contributes about 30 pF to each line, so the permitted extra capacitance to ground is 50 pF.

US 3,946,738 A discloses a leakage current cancelling circuit for use with electrosurgical instrument. EP 1 854 423 A2 refers to a system for reducing leakage current in an electrosurgical generator. US 2019/ 274717 A1 discloses methods for controlling temperature in ultrasonic device.

The objective of the invention is to reduce leakage currents caused by a leakage capacitance of a direct current DC isolated electrical radiofrequency (RF) or microwave frequency device.

SUMMARY OF THE INVENTION

It is an aim of the subject matter of the independent claims to solve this objective. Preferred embodiments of the invention are defined in the dependent claims.

At its most general, the present invention provides the connection of a series connected inductor and capacitor between one line of the RF and/or microwave bipolar feed and ground or neutral.

According to a first aspect of the invention, there is provided an energy conveying device for conveying radiofrequency electromagnetic energy and/or microwave electromagnetic energy. The energy conveying device comprises a first conductive line and a second conductive line configured to convey a radiofrequency electromagnetic energy and/or microwave electromagnetic energy having a first frequency, an electrical element providing a first leakage capacitance, and a tuning circuit including an inductor unit and a capacitor unit having a ground capacitance. The first leakage capacitance is between the first conductive line and a ground and/or between the second conductive line and the ground. The inductor unit and the capacitor unit are connected (or coupled) in series between the ground and either the first conductive line or the second conductive line. The inductor unit has a tuning inductance such that the tuning circuit and the first leakage capacitance form a resonant circuit having a resonance frequency (or resonant frequency) that is aligned with the first frequency.

According to a second aspect of the invention, there is provided a generator device for generating and/or conveying radiofrequency electromagnetic energy and/or microwave electromagnetic energy, comprising a generator unit configured to generate radiofrequency electromagnetic energy and/or microwave electromagnetic energy of the first frequency, and the energy conveying device. The energy conveying device is electrically coupled (such as directly or indirectly connected) to the generator unit.

According to a third aspect of the invention, there is provided an electrosurgical apparatus, comprising the generator device and a radiating device for emitting radiofrequency electromagnetic energy and/or microwave electromagnetic energy.

The invention provides a reliable and effective reduction or a substantially complete cancellation of leakage currents.

The term "electrosurgical" is used in relation an instrument, apparatus, or tool which can be used during surgery and which utilises radio frequency (RF) and/or microwave electromagnetic (EM) energy to treat a treatment zone. Herein, "microwave EM energy" may mean electromagnetic energy having a (stable fixed) frequency in the range 300 MHz to 100 GHz, preferably in the range 1 GHz to 60 GHz. Preferred spot frequencies for the microwave EM energy include 915 MHz, 2.45 GHz, 5.8 GHz, 14.5 GHz, 24 GHz. 5.8 GHz may be preferred. Radiofrequency (RF) may mean RF energy having a (stable fixed) frequency in the range 20 kHz to around 1 GHz. 400 kHz may be preferred.

In use, the treatment zone may include biological tissue in a patient's lungs, uterus, gastrointestinal tract or other organs. Microwave energy can cause dielectric heating in biological tissue, which can be used to ablate tissue in a localised volume around the radiating device. Therefore, by inserting the radiating device directly into a treatment zone, including e.g. a tumour, lesion or fibroid, microwave energy can be applied to tissue in the treatment zone in order to ablate it. Thus, microwave EM energy carried by the energy conveying device may be radiated from the radiating device into a treatment zone, in order to coagulate and/or ablate the treatment zone. RF energy may be used to cut tissue in the treatment zone.

The radiating device may include an RF and/or microwave antenna. The antenna may be a conventional monopole antenna formed on the end of the energy conveying device. The energy conveying device may include a (coaxial) transmission line, e.g. a conventional coaxial cable. An inner conductor of the coaxial cable may be connected to the radiating device or the antenna from which RF and/or microwave energy can radiate. The radiating device may include one or more dielectric materials to provide dielectric loading of the antenna, in order to enhance or shape the energy emission profile of the microwave antenna. The coaxial feed cable may include an outer conductor which is separated from the inner conductor by a dielectric material.

The radiating device may include one or more electrodes (e.g. two electrodes) at least one of which is connected or coupled to the first conductive line or the second conductive. For example, a first electrode is connected or coupled to the first conductive line and a second electrode is connected or coupled to the second conductive line. The electrodes may be spaced from each other to allow the placement of tissue there between. Supplying RF energy may result in cutting the tissue between the electrodes.

The electrosurgical apparatus can be used to apply RF and/or microwave energy to matter in the vicinity of the radiating device, such as biological tissue, fluids or other materials.

The electrosurgical apparatus may be an endoscopic electrosurgical apparatus. For example, the electrosurgical apparatus may be sized and shaped to be configured to be inserted in a scoping device. The scoping device can be used to guide the radiating device of the electrosurgical apparatus to the treatment zone, i.e. that the radiating device is placed close to the treatment zone. The radiating device may exit the scoping device during use of the electrosurgical apparatus, e.g. the radiating device protrudes from a distal end of the scoping device.

The generator device may include means for generating and/or conveying RF electromagnetic energy and/or microwave EM energy. The generator unit may be configured to generate electromagnetic radiation of a fixed single frequency or of a plurality of fixed single frequencies. Alternatively or additionally, the generator unit may be tuneable to generate electromagnetic radiation of various frequencies, for example in a continuous range of frequencies between a minimum frequency and a maximum frequency. The generator unit may be connected to a power supply which provides the energy for generating the RF electromagnetic energy and/or microwave EM energy.

The generator unit is electrically and/or electronically (directly or indirectly) connected to the energy conveying device. Preferably, the generator unit generates the RF EM energy and/or microwave EM energy which is conveyed by the energy conveying device to the radiating device where the RF EM energy and/or microwave EM energy is radiated into the treatment zone.

The first conductive line and/or the second conductive line may be provided for conveying energy. In this embodiment, the first and second conductive line can be connected to the generator unit. Alternatively, one of the first conductive line and the second conductive line may be grounded or connected to neutral. In this embodiment, the one of the first conductive line and a second conductive line that is grounded may not be electrically connected to the generator unit.

The first conductive line and/or the second conductive line may include wires that are electrically isolated from each other (e.g. a twisted pair cable). As discussed above, the first conductive line and/or the second conductive line may form coaxial transmission line. Thereby, an inner conductor is arranged coaxially with an outer conductor and are separated from each other by a dielectric layer. The outer conductor may completely surround the inner conductor in a circumferential direction.

The first conductive line and/or the second conductive line may be configured to convey electromagnetic radiation in a range of frequency from a minimum frequency to a maximum frequency. However, the first conductive line and/or the second conductive line are configured to convey electromagnetic radiation of a first frequency which can be the frequency that is generated by the generator unit.

The energy conveying device may be electrically isolated from the ground or neutral when subjected to constant voltage potentially resulting in a direct current (DC), for example during a test routine. Thus, when the energy conveying device is subjected to a constant voltage (or DC operation), no leakage current occurs.

The electrical element does not provide a leakage current during a DC operation, i.e. if a current of the frequency zero was conveyed by the energy conveying device. This means that the electrical element is also isolated to the ground or neutral in a theoretical DC operation of the energy conveying device.

The energy conveying device, the generator unit, and/or the generator device can be DC isolated, i.e. no leakage currents are present when subject to a constant voltage which could result in a DC current.

However, the electrical component causes a leakage current during an alternate current (AC) operation of the energy conveying device, such as when conveying RF energy or microwave energy. This is because the electric element acts as, functions as, or has (provides) the characteristics of a capacitor (a first leakage capacitor having the first leakage capacitance) between the first conductive line and/or the second conductive line and the ground/neutral (these characteristics of the capacitance provide DC isolation). It is noted that the first leakage capacitor does not necessarily refer to an actual capacitor component that is or is part of the electrical component, but instead to any circuit or circuit element that functions as (e.g. provides the characteristics of) a capacitor during use. That is, the first leakage capacitor may be provided by a transformer, multiplexer, or some other electric component which is not a capacitor component. Of course, the first leakage capacitor could also be or include a capacitor component. In any case, the first leakage capacitor may be represented in a circuit diagram of the energy conveying device to illustrate the first leakage capacitance provided by the electrical element or the energy conveying device.

The electrical element may provide further capacitor characteristics. However, for this description, only those capacitor characteristics are relevant which occur between the first/second conductive line and the ground/neutral and which interact with the conveyed EM energy at the first frequency to cause a detectable, non-negligible, significant or relevant leakage current. Thus, negligible first leakage capacitances (between the first/second conductive line and the ground/neutral) which do not significantly interact with the first frequency may not be considered in this invention. For example, negligible leakage currents caused by further capacitances of the electrical element which provide a power in a 200 $\Omega$ (non-inductive) test resistor less than 0.1 %, 0.05 %, or 0.01 % of the maximum bipolar rated output power (according to BS EN 60601-2-2:2009 - see above) at the first frequency may not be considered in this invention. Those negligible first leakage capacitances or negligible leakage currents are present but can be considered acceptable, or their effect can be considered negligible. In other words, without the tuning element, the non-negligible first leakage capacitance that is the subject of this invention causes a non-negligible leakage current (or a power in a 200 $\Omega$ (non-inductive) test resistor) at the first frequency that is greater than 0.1 %, 0.05 %, or 0.01 % of the maximum bipolar rated output power (according to BS EN 60601-2-2:2009 - see above). If those non-negligible first leakage capacitances or non-negligible leakage currents are present, they are considered unacceptable, and their effect can be considered significant. Hence the present invention aims to reduce or substantially cancel out the sum of such non-negligible and negligible leakage capacitances or the sum of the non-negligible and negligible leakage currents. It is noted that unless otherwise stated references herein to "leakage current" or "leakage capacitance" should be understood as "the sum of non-negligible and negligible leakage capacitances" and "the sum of the non-negligible and negligible leakage currents", respectively. However, it is also noted that the above defined references to "leakage current" or "leakage capacitance" may effectively refer to "non-negligible leakage current" and "non-negligible leakage capacitance", respectively, since the negligible leakage capacitances or the negligible leakage currents may not be problematic. In other words, the invention aims to reduce or cancel out an unacceptable or non-negligible sum capacitances (the sum of all leakage capacitances which can be represented by the first leakage capacitor having the first leakage capacitance) and/or sum leakage currents (the sum of all leakage currents) to a value which is negligible or acceptable.

The term "electrical element" needs to be understood as any electrical component (e.g. circuit component) and/or any combination of interconnected electrical components (e.g. circuit or circuits) of the energy conveying device that provide the first leakage capacitance between the first conductive line and a ground/neutral and/or between the second conductive line and the ground/neutral during AC operation (resulting in the leakage current) and provides electrical isolation between the first conductive line and a ground/neutral and/or between the second conductive line and the ground/neutral during the during DC operation.

Thus, leakage currents are intended to be avoided but are inherently present due to the interaction of some of the electrical components of the energy conveying device with each other or the ground/neutral. "Leakage" may be considered as the unintended and/or unwanted transfer of electrical energy across a boundary normally viewed as insulating. "Leakage" may also mean an unwanted transfer of energy from one circuit to another.

As such, the electrical element may be any component and/or any combination of interconnected electrical components of the energy conveying structure that provides a leakage capacitance (resulting in a leakage current). The electrical element may affect only the first conductive line, only the second conductive line, or both the first conductive line and the second conductive line.

The electrical element may be permanently connected or electrically coupled to the first conductive line and/or the second conductive line. The electrical element may also be configured to be selectively connected and dis-connected or electrically coupled and dis-coupled to the first conductive line and/or the second conductive line, for example using a switch. The electrical element may include a replaceable element that can be connected to the energy conveying structure, such as a tool or the radiating device.

For example, the electrical element may include an isolation transformer, a microwave choke, and/or a multiplexer.

The isolation transformer (or output transformer) may transfer the generated RF energy and/or microwave energy onto the energy conveying device or between sections of the energy conveying device. The isolation transformer (or output transformer) may be coupled or connected between the generator unit (e.g. a first generator and/or a second generator, respectively) and the first conductive line and/or a second conductive line. Alternatively, the isolation transformer (or output transformer) may be coupled or connected between sections of the first conductive line and/or a second conductive line. The isolation transformer can be provided for electrical isolation of the first conductive line and/or a second conductive line (or sections thereof) from the power supply of the generator unit, e.g. from the power supply of the first generator and/or the second generator.

It is also possible that the isolation transformer is provided between the generator unit and the first conductive line and/or the second conductive line. For example, each generator of the generator unit (first generator, second generator, etc) is provided with a respective isolation transformer. Thus, connecting the first generator to the energy conveying device (for example using or via a multiplexer) changes the leakage capacitance due to the leakage capacitances of the isolation transformer and the first generator compared to a connection state in which the second generator is connected to the energy conveying device.

The isolation transformer may be a transformer used to transfer electrical power from a source of alternating current (AC) power (e.g. the generator unit) to some equipment or device (e.g. the energy conveying device) while isolating the powered device from the power source, such as for safety reasons. The isolation transformer may provide galvanic isolation; no conductive path is present between source and load. This isolation can be used to protect against electric shock, to suppress electrical noise, and/or to transfer power between two circuits which must not be connected. The isolation transformer can be built with special isolation between primary and secondary circuits and can be specified to withstand a high voltage between windings. The isolation transformer may include two coils or windings one of which is connected to the first conductive line and/or the second conductive line. This coil or winding may provide the first leakage capacitance.

The outer conductor of a coaxial transmission line can be important for preventing microwave leakage. Introducing a break in the outer conductor can require a device called a microwave choke to prevent leakage. This interruption in the outer conductor acts or functions as a leakage capacitor. This structure of the microwave choke may act as a leakage capacitor because, for isolating microwave chokes, low capacitance and low leakage tend to be conflicting targets.

The multiplexer may be a diplexer and can combine the input from various sources into one output. For example, a multiplexer (or diplexer) is used to combine the output of three or more (two) generators to a single output which is connected or coupled to the first conductive line and/or the second conductive line.

The different connection states of the multiplexer may result in an increase or change in the leakage capacitance since different generators and/or isolation transformers are connected to the energy conveying device. In other words, the combination of the multiplexer, the generator unit, and/or the isolation transformer can be regarded as an electrical component whose leakage capacitance is variable depending on the connection state of the multiplexer. Thus, the electrical element may (alternatingly) provide the first leakage capacitance and a second leakage capacitance.

The inductor unit may include one or more inductors and/or other electrical components that provide an inductance. The combined inductance of the components of the inductor unit provides the tuning inductance. Similarly, the capacitor unit includes one or more capacitors and/or other electrical components that provide a capacitance. The combined capacitance of the electrical components of the capacity unit provides a ground capacitance.

The inductor unit may also include one or more tuning capacitors providing a tuning capacitance of the inductor unit.

The capacitor unit provides the DC isolation of the tuning circuit with regards to the first conductive line or the second conductive line. Thus, the capacitor unit may be regarded as a DC isolator. Optionally, the capacitor unit is electrically connected between the inductor unit and the ground/neutral or vice versa. Further optionally, the inductor unit may be connected between the capacitors of the capacitor unit.

The tuning circuit and the first leakage capacitor/capacitance are arranged such that the tuning circuit and the first leakage capacitor/capacitance form a parallel resonant circuit or LC circuit. This means, that both the tuning circuit and the first leakage capacitor/capacitance are connected in parallel between the ground/neutral and the first conductive line and/or the second conductive line.

The following formula describe the impedance of a parallel resonant circuit:

$$Z(\omega) = -j\left(\frac{1}{C}\right)\left(\frac{\omega}{\omega^2 - \omega_0^2}\right) \qquad (1)$$

$\omega_0$ is the angular resonance frequency of the resonant circuit ($\omega_0 = 2\pi f_0$, wherein $f_0$ is the resonance frequency):

$$\omega_0 = \frac{1}{\sqrt{LC}} \qquad (2)$$

$C$ is the first leakage capacitance. $L$ is the tuning inductance corrected by ground capacitance. $\omega$ is the angular first frequency which can be the angular frequency that is provided by the generator device.

A more accurate description of the angular resonance frequency is given in following equation (3):

$$\omega_0^2 = \frac{1}{LC} + \frac{1}{LC_s} = \frac{1}{L}\left(\frac{1}{C} + \frac{1}{C_s}\right) \qquad (3)$$

$C$ is the first leakage capacitance, $L$ is the tuning capacitance, and $C_S$ is the ground capacitance. Thus, L of equation (2) is effectively smaller than the tuning inductance L as apparent from equation (3).

It is apparent from equation (1) that the impedance of the resonant circuit becomes greater the more the first frequency $\omega$ approaches the resonance frequency $\omega_0$ of the resonant circuit. Higher impedance means that less leakage current flows through the first leakage capacitor. Thus, tuning or setting the tuning impedance, the tuning capacitance, and/or the ground capacitance according to equation (2) such that $\omega_0 \approx \omega$ results in an effective reduction or even a substantial cancellation of leakage currents through the electrical component.

Thus, the alignment of the resonance frequency of the resonant circuit with the first frequency means that the resulting impedance of the resonant circuit becomes high so that the leakage current is reduced to an acceptable level or a level as defined by standards and/or industrial norms. For example, the power due to leakage current (or a power in a 200 Ω (non-inductive) test resistor) at the first frequency is less than 1 %, 0.75 %, 0.5 %, 0.25 %, or 0.01 % of the maximum bipolar rated output power (according to BS EN 60601-2-2:2009 - see above). For example,

$$\left| f - f_0 \right| \leq 1 \text{ kHz}$$

5 kHz, or 10 kHz. Alternatively,

$$\left| f - f_0 \right| / f_0$$

may be smaller than 0.25 %, 1.25 %, or 2.5 %, wherein f is the first frequency and $f_0$ is the resonance frequency of the resonant circuit.

The first leakage capacitance may be measured and the tuning inductance, the ground capacitance, and/or the tuning capacitance set or tuned/varied/changed accordingly. Alternatively or additionally, the leakage current is monitored (via the 200 Ω test resistor) and the tuning inductance, the ground capacitance, and/or the tuning capacitance is varied for reducing the leakage current. This embodiment does not require an explicit measurement of the leakage current.

In summary, the purpose of the inductor unit/tuning circuit is to create a parallel resonant circuit with the first leakage capacitance, to increase the total impedance to a very high value, resulting in a considerable reduction in the effective leakage capacitance, and a reduction in the power in the 200 Ω test resistor connected between the other line (the line of the first conductive line and the second conductive line to which the electrical element is not connected) and ground or neutral.

The ground capacitance of the capacitor unit is greater than the first leakage capacitance of the first leakage capacitor and/or the tuning capacitance, optionally by at least a factor of 10, 100, or 1000. For example, the tuning capacitance is of the order of 1 μF.

If the ground capacitance is significantly larger than the first leakage capacitance and/or the tuning capacitance, the capacitor unit hardly affects the impedance of equation (1). Instead, the capacitor unit provides the DC isolation of the tuning circuit but can be essentially disregarded for the calculation of the impedance according to equation (1). This is due to the fact that a very large ground capacitance does not interact or hardly interacts with the first frequency. For example for an operation at 400 kHz, the ground capacitance of the order of 1 μF in series with an inductor unit having a tuning inductance of the order of 1 mH would be suitable for cancelling a first leakage capacitance of the order of 100 pF.

A further view on this is that the resonant circuit solely formed by the inductor unit and the capacitor unit has a resonance frequency that is significantly offset from the first frequency such that this resonant circuit can be disregarded close to the first frequency. The resonance frequency of the resonant circuit solely formed by the inductor unit and the capacitor is offset from the first frequency due to the large ground capacitance.

In an optional embodiment, the inductor unit includes one tuning inductor having the tuning inductance or two or more tuning inductors permanently connected or coupled to each other for commonly providing the tuning inductance.

In this embodiment, the tuning inductance is fixed which is suitable if the generator device generates a fixed first frequency or the generator device generate frequencies close the first frequency, for example ±3%, ±5%, or 110% offset from the first frequency. In the latter case, the tuning circuit can be still effective in reducing leakage currents.

Further, the first leakage capacitance may also vary for different tools used with the generator device and/or the energy conveying device, and/or for different microwave generators attached to the RF generator device. These changes may only slightly vary the first leakage capacitance so that the leakage current can still effectively reduced by the tuning circuit.

The provision of two or more tuning inductors allows to set the tuning inductance using inductors having predetermined frequencies. For example, by connecting or coupling the tuning inductors in series and/or in parallel, the tuning inductance can be appropriately set.

Further, the inductor unit may include one or more tuning capacitors for setting the resonance frequency of the resonant circuit to the first frequency; this is equal to changing the resonance frequency of the tuning circuit. Thus, the one or more tuning capacitors provide a different means for changing the resonance frequency of the resonant circuit if, for example, an adequate setting of the resonance frequency cannot be achieved by setting the tuning inductance alone.

In an optional embodiment, the inductor unit includes two or more tuning inductors and a switch unit, the switch unit being configured to selectively electrically connect or couple one or more tuning inductors to the capacitor unit for selectively changing the tuning inductance of the tuning circuit. Alternatively, the inductor unit includes at least one tuning inductor, at least one tuning capacitor, and a switch unit, the switch unit being configured to selectively electrically connect one or more tuning inductors and/or one or more tuning capacitors to the capacitor unit for selectively changing the resonance frequency of the resonant circuit (or the tuning circuit which is an LC circuit where the inductor unit and the capacitor unit are connected in series).

In the above embodiments, the switch unit is used for connecting or disconnecting one or more tuning inductor and/or one or more tuning capacitors to or from the tuning circuit, in particular the tuning inductor that is permanently connected to the capacitor unit. In this way, the tuning inductance or the resonance frequency of the resonant circuit can be switched between two or more values.

The switch unit may include one or more electrical switches. In a first state of the switch, the one or more of tuning capacitors and/or one or more of the tuning inductors are connected/coupled in series to the capacitor unit (and in parallel or in series with the tuning inductor that is permanently (even in a second state of the switch) connected/coupled in series with the capacitor unit). In the second state of the switch, the one or more of tuning capacitors and/or one or more of the tuning inductors are not connected/coupled to the capacitor unit. Thus, the one or more of tuning capacitors and/or one or more of the tuning inductors are not part of the resonant circuit. In short, in the second state of the switch, the number of electrical components contributing to the tuning circuit differs from the number when the switch is in the first state. As a consequence, the tuning inductance and/or the resonance frequency of the resonant circuit differs depending on whether the switch is in the first state or the second state. The first state and the second state may correspond to an open state and closed state, respectively, or vice versa. For example, in a closed state, the switch provides a bypass to a tuning inductor and/or tuning capacitor.

The more switches are provided, the more resonance frequencies can be selected by selectively open or closing the respective switches.

In an optional embodiment, the first conductive line and the second conductive line are configured to convey radiofrequency electromagnetic energy and/or microwave electromagnetic energy having a second frequency which is different to the first frequency and/or the electrical element provides a second leakage capacitance which is different to the first leakage capacitance, wherein optionally the tuning circuit is configured to selectively change the resonance frequency of the resonant circuit such that the resonance frequency is aligned with the first frequency or the second frequency and/or the tuning circuit is configured to selectively change the resonant circuit such that the tuning circuit and the first or second leakage capacitances form a resonant circuit having respective resonance frequencies (e.g. first and second resonance frequencies) that are aligned with the first frequency.

The alignment of the resonance frequency of the resonant circuit with the second frequency and/or the second leakage capacitance means that the resulting impedance of the resonant circuit becomes high so that the leakage current is reduced to an acceptable level or a level as defined by standards and/or industrial norms. For example, the power due to leakage current (or a power in a 200 Ω (non-inductive) test resistor) at the second frequency is less than 1 %, 0.75 %, 0.5 %, 0.25 %, or 0.01 % of the maximum bipolar rated output power (according to BS EN 60601-2-2:2009 - see above). For

example,

$$\left| f - f_0^{\square} \right| \leq 10 \text{ Hz},$$

50 Hz, or 100 Hz. Alternatively,

$$\left| f - f_0^{\square} \right| / f$$

may be smaller than 1 %, 3 %, 5 %, or 10 %, wherein f is the second frequency and $f_0$ is the resonance frequency of the resonant circuit.

The second frequency may correspond to a second frequency that is generated by the generator unit. The generator unit may be configured to alternatively generate either the first frequency or the second frequency. For example, the generator unit may include an input device or interface for selecting the first frequency or the second frequency to be generated.

Depending on the selection of the first frequency or the second frequency, the switch unit or a single switch is either set to the first state or the second state. In first state, the tuning inductance or the resonance frequency of the resonant circuit are aligned with first frequency. In second state, the tuning inductance or the resonance frequency of the resonant circuit are aligned with the second frequency.

Optionally, the generator unit may be configured to generate a third, a fourth, etc frequency and the first conductive line and the second conductive line are configured to convey a radiofrequency electromagnetic energy and/or microwave electromagnetic energy having the third, a fourth, etc frequency. In this case, the switch unit may include further switches for selectively setting the tuning capacitance or the resonance frequency of the resonant circuit to the third, a fourth, etc frequency using the principles outlined above.

In a similar manner, the connection or disconnection of one or more different electrical elements to the energy conveying structure (such as a generator), the replacement of the electrical component (such as the replacement of the radiating device), and/or internal changes of the electrical component (such as different settings) may change the leakage capacitance such that the first leakage capacitance changes to the second leakage capacitance. The same features, characteristics, and/or functionalities as described in conjunction with the first leakage capacitance equally apply to the second leakage capacitance. In particular, the second leakage capacitance may be represented by a second leakage capacitor.

The second leakage capacitance may be present instead of or in addition to the first leakage capacitance. Further, an additional leakage capacitance may be electrically connected to the first leakage capacitance providing the second leakage capacitance. The change in the leakage capacitance requires a change in the tuning circuit similar to the change in view of the first and the second frequencies.

In other words, a change from the first leakage capacitance to the first leakage capacitance changes the resonance frequency of the resonant circuit from a first resonance frequency to a second resonance frequency. The second resonance frequency of the resonant circuit may no longer be aligned with the first frequency that is conveyed in the energy conveying device. The tuning circuit may then need to be re-set or re-tuned such that the second resonance frequency is aligned with the first frequency. For example, the power due to leakage current caused by the second leakage capacitance (or a power in a 200 $\Omega$ (non-inductive) test resistor) at the first frequency is less than 1 %, 0.75 %, 0.5 %, 0.25 %, or 0.01 % of the maximum bipolar rated output power (according to BS EN 60601-2-2:2009 - see above). For example,

$$\left| f - f_0^{\square} \right| \leq 10 \text{ Hz},$$

50 Hz, or 100 Hz. Alternatively,

$$\left| f - f_0^{\square} \right| / f$$

may be smaller than 1 %, 3 %, 5 %, or 10 %, wherein f is the first frequency and $f_0$ is the second resonance frequency of the resonant circuit.

It is also possible that a change in the frequency that is conveyed in the energy conveying device (e.g. a change from the first frequency to the second frequency) and a change in the leakage capacitance (e.g. a change from the first leakage capacitance to the second leakage capacitance resulting in a change from the first resonance frequency to the second resonance frequency) occurs at the same time. In this case, the second frequency is aligned with the second resonance frequency.

In an optional embodiment, the inductor unit includes at least one tuning inductor and/or at least one tuning capacitor that are tuneable or variable for changing the resonance frequency of the tuning circuit.

In a further optional embodiment, the first conductive line and the second conductive line are configured to convey the radiofrequency electromagnetic energy and/or microwave electromagnetic energy having the second frequency and/or the electrical element provides a second leakage capacitance which is different to the first leakage capacitance, wherein optionally the tuning circuit is configured to be tuned or varied to selectively change the resonance frequency of the resonant circuit such that the resonance frequency is aligned with the first frequency or the second frequency and/or the tuning circuit is configured to selectively change the resonant circuit such that the tuning circuit and the first or second leakage capacitances form a resonant circuit having respective resonance frequencies (e.g. first and second resonance frequencies) that are aligned with the first frequency.

A change in the resonance frequency of the tuning circuit equals a change in the resonance frequency of the resonant circuit since it is assumed that the first leakage capacitance is constant. In other words, the resonant circuit includes the tuning circuit and the first leakage capacitance.

The tuneable tuning inductor is configured to vary or change its inductance and the tuneable tuning capacitor is configured to vary or change its capacitance. In other words, the tuning or varying is executed on the tuning inductor and/or the tuning capacitor itself and no switch external to the tuning inductor and/or the tuning capacitor is employed. Thus, tuning or varying the tuning inductance of the tuning inductor and/or the tuning capacitance of the tuning capacitor changes the resonance frequency of the resonant circuit. Optionally, a continuous or stepwise change in the tuning inductance of the tuning inductor and/or the tuning capacitance of the tuning capacitor is possible. The tuneable tuning inductor and the tuneable tuning capacitor may be considered as a variable tuning inductor (or a tuning inductor having a variable/tuneable inductance) and a variable tuning capacitor (or a tuning capacitor having a variable/tuneable capacitance), respectively.

The tuneable tuning inductor may include "'Slot Ten' 1mm Tunable Inductors" by Coilcraft Inc. The tuneable tuning capacitor may include "Ø 7.5 mm Film Dielectric Trimmers" by Vishay Intertechnology, Inc.

This allows a match or approximation of the resonance frequency according to equations (2) or (3) to the frequency that is generated by the generator device, especially if the generator device allows the generation of frequencies in a continuous range of frequencies.

In an optional embodiment, the energy conveying device further comprises a tuneable or variable additional capacitor unit having an additional capacitance, wherein the additional capacitor unit is connected in series between the ground and either the first conductive line or the second conductive line.

The additional capacitor unit and the tuning circuit are connected in parallel between the ground and either the first conductive line or the second conductive line. The additional capacitor unit may include one or more capacitors connected in series. For example, a first additional capacitor may be tuneable and a second additional capacitor may not be tuneable (i.e. having a fixed capacitance). The features, characteristics, and/or embodiments of the tuneable inductor described above may equally apply to the tuneable additional capacitor unit. The provision of two more capacitors with the additional capacitor unit may be done for redundancy reasons.

The additional capacitor unit can be considered changing the resonance frequency of the resonant circuit and/or changing the resonance frequency of the tuning circuit. Thus, the additional capacitor unit can be used instead or in addition to the tuneable inductor unit for varying the resonance frequency.

The values required for variation of the capacitance can be quite small, because the leakage capacitance can be quite small. For example, the additional capacitance is in the same order of magnitude as the first or second leakage capacitances. This can be suitable for variable or tuneable capacitors.

In an optional embodiment, the capacitor unit may have the features and characteristics of the additional capacitor unit. One of the capacitors of the capacitor unit may be tuneable or variable. Thus, capacitor unit includes two or mor capacitors and the ground capacitance may be variable or tuneable by tuning/varying the tuneable/variable one(s) of the capacitors of the capacitor unit. The tuneable/variable one(s) of the capacitors of the capacitor unit may have the features and/or characteristics of the (tuneable) first additional capacitor of the additional capacitor unit. The variation of the ground capacitance results in a variation of the resonance frequency of the tuning circuit as apparent from the equations provided above. The variable ground capacitance may be provided in addition or as an alternative to the above-described embodiments for changing the resonance frequency of the tuning circuit.

In an optional embodiment, the first conductive line and the second conductive line are configured to convey the radiofrequency electromagnetic energy and/or microwave electromagnetic energy having a second frequency which is different to the first frequency, and/or the electrical element provides a second leakage capacitance which is different to the first leakage capacitance. The additional capacitor unit is optionally configured to be varied to selectively change the resonance frequency of the resonant circuit such that the resonance frequency is aligned with the first frequency or the second frequency and/or the additional capacitor unit is configured to selectively change the tuning circuit such that the tuning circuit and the first or second leakage capacitances form a resonant circuit having respective resonance frequencies that are aligned with the first frequency.

Thus, the additional capacitor unit can be used to change the effective inductance L of equation (2). Analogous features, characteristics, and/or embodiments as described with the tuneable inductor unit apply to the tuneable additional capacitor unit.

In an optional embodiment, the energy conveying device further comprises a measuring device for measuring and/or monitoring a leakage current induced by the first leakage capacitance.

The measuring device may include any means for determining a leakage current and/or the loss of power due to leakage. For example, the measuring device determines the current power consumption and compares the current power consumption to a predetermined power consumption. This comparison may be indicative of a loss of power due to leakage. Further, the measuring device may include an electric circuit for directly measuring a leakage current. For example, the current flowing to the ground (leakage current) is measured by connecting the meter of the measuring device (such as a galvanometer) in series with a ground connection of the energy conveying device. The measuring device may include a leakage current transformer. In a further embodiment, the measuring device includes the 200 Ω test resistor and a current meter or galvanometer for measuring the current flowing through the 200 Ω test resistor.

The leakage current can be measured using the measuring device (e.g. a current transformer or a galvanometer) or by measuring the voltage across the test resistor and using the formula I=V/R, wherein I is the current flowing through the test resistor, R is the resistance of the test resistor, and U is the voltage drop over the test resistor. The test resistor is mainly there for conformity testing. In normal use, a test resistor may not be connected to the energy conveying structure. The test resistor is there to replace the human body, which may provide this kind of resistive path to earth or neutral, and should not have a high leakage current passing through it.

Using a test resistor may be helpful during set-up, as the test resistor will not be in place during use. However, the procedures described here and below may be used to set up an automatic library of settings for each device or a set-up to be used, to permit switching/tuning to the pre-determined minimum leakage current configuration.

In an optional embodiment, the energy conveying device further comprises a display device for displaying the leakage current measured by the measuring device and/or an input device for changing the resonance frequency of the resonant circuit by tuning the at least one tuning inductor and/or the at least one tuning capacitor.

This embodiment refers to a manual tuning of the tuning circuit or a manual reduction of the leakage current. The display device may include a display and/or screen and is electrically and/or electronically connected/coupled to the measuring device. The display device displays the leakage current as measured or determined by the measuring device. Thus, the measured or determined leakage current is displayed to a user.

The input device allows changing or varying the resonance frequency of the tuning circuit and, thus, of the resonant circuit. The input device may include one or more buttons, dials, and/or levers which are operatively connected to the tuning inductor and/or the tuning capacitor. If the user operates the input device, the resonance frequency of the tuning circuit is changed. Thus, an operation of the input device results in a change of the leakage current. The leakage current can be minimised by appropriately operating the input device and, thus, appropriately setting the resonance frequency of the tuning circuit.

In an optional embodiment, the energy conveying device further comprises a control device configured to tune and/or vary the tuning inductance of the at least one tuning inductor and/or the tuning capacitance of the at least one tuning capacitor to change the resonance frequency of the resonant circuit based on the leakage current measured by the measuring device.

This embodiment refers to an automatic tuning of the tuning/resonant circuit or an automatic reduction of the leakage current. The control device may include a processor and a memory on which an algorithm or programme is stored that is executed by the processor. The control device may include a computer or the like. The control device may include an actuator, such as an electric motor, for tuning the inductor unit (such as the tuning inductor and/or the tuning capacitor). The actuator may be controlled by the processor. Further, the control device is electrically or electronically connected to the measuring device for receiving signals indicative of the leakage current. The program, method, or algorithm stored on the memory and executed by the processor minimises or reduces the leakage current based on the measurements received from the measuring device.

The control device is electronically and/or electrically connected to the tuning circuit for changing the resonance frequency of the tuning circuit. For example, the actuator of the control device is connected to the input device for changing the resonance frequency of the tuning circuit. The control device changes the resonance frequency of the tuning circuit such that leakage current is reduced or minimised.

In an optional embodiment, the capacitor unit comprises two or more capacitors, optionally connected together in series.

At least two capacitors are connected in series between the inductor unit and the ground/neutral. The second, third, etc capacitors can be provided to give redundancy in case of short circuit of the first capacitor, as the capacitor unit can be a safety critical component for DC isolation.

In an optional embodiment, the energy conveying device further comprises a second tuning circuit including a second inductor unit and a second capacitor unit having a second ground capacitance, the inductor unit and the capacitor unit being connected in series between the ground and one of the first conductive line and the second conductive line, the second inductor unit and the second capacitor unit being connected in series between the ground and the other one of the first conductive line and the second conductive line, wherein optionally the second inductor unit has a second tuning inductance such that the second tuning circuit and the first leakage capacitor form a resonant circuit having a resonance frequency that is aligned with the first frequency.

The second tuning circuit may be provided for reducing leakage currents caused by the same electrical element or further electrical element however affecting the other one of the first conductive line and the second conductive. In a further embodiment, the electrical element may provide a first leakage capacitance for the first conductive line and the second conductive line. In this case, the second tuning circuit reduces or eliminates leakage currents from the other one of the first conductive line and the second conductive line.

The same considerations, optional embodiments, and/or characteristics as discussed in conjunction with the (first) tuning circuit equally applies to the second tuning circuit.

Further, two or more first and/or second tuning circuits may be provided, each of the two or more first and/or second tuning circuits is configured to reduce a leakage current caused by a respective electrical element. Thus, if several electrical elements are present causing several different leakage currents, an equal number of tuning circuits may be provided for reducing or minimalizing the respective leakage currents.

In an optional embodiment, the generator unit comprises a first generator configured to generate radiofrequency electromagnetic energy and/or microwave electromagnetic energy having the first frequency, a second generator configured to generate radiofrequency electromagnetic energy and/or microwave electromagnetic energy having the second frequency which is different to the first frequency, and a multiplexer including a first input port, a second input port, and an output port, wherein the first generator is connected to the first input port, the second generator is connected to the second input port, and the energy conveying device is connected to the output port.

The first generator and the second generator may be generators providing a respective fixed frequency or may be tuneable generators which are configured to generate a frequency in a continuous range between a minimum frequency and a maximum frequency. The first frequency and the second frequency can differ from each other.

The generator unit may include a generator for generating microwave frequency and a microwave-to-RF converter. RF frequency may be generated by coupling the microwave-to-RF converter to the microwave frequency generator, for example by using a switch. The connection of the microwave-to-RF converter to the microwave frequency generator may change the first leakage capacitance.

In one embodiment, the multiplexer is a diplexer. However, the invention is not limited thereto. The generator unit may include n generators (first to n-th generators) and the multiplexer n inputs ports (first to n-th input ports), wherein n is an integer. The k-th generator is connected to the k-th input port, wherein k is an integer fulfilling $1 \leq k \leq n$.

The output port supplies the electromagnetic energy supplied to each input port to the output port and, therefore, combines the output of each generator to the energy conveying device. The first to n-th generator may operate simultaneously or alternatingly.

In an optional embodiment, the electrosurgical apparatus further comprises an elongate body configured to be inserted in a working channel of a surgical scoping device and including a proximal and a distal end, wherein the radiating device is attached to the distal end, and wherein the first conductive line and the second conductive line extend in the elongate body.

The elongate body may be a cover of the first conductive line and a second conductive line or surround the first conductive line and a second conductive line. The elongate body may include a tube or hose. The first conductive line and a second conductive line may be flexible to be inserted into the working channel of the scoping device.

Also disclosed herein is a method for reducing leakage current with an energy conveying device for conveying radiofrequency electromagnetic energy and/or microwave electromagnetic energy, the energy conveying device comprising a first conductive line and a second conductive line, the method comprising the steps of: (i) supplying radiofrequency electromagnetic energy and/or microwave electromagnetic energy having a first frequency to the first conductive line and/or a second conductive line, (ii) providing a tuning circuit including an inductor unit and a capacitor unit having a ground capacitance, the inductor unit and the capacitor unit are connected in series between a ground and the first conductive line and/or between the ground and the second conductive line, and (iii) setting a tuning inductance of the inductor unit such that the tuning circuit and the first leakage capacitance form a parallel resonant circuit having a resonance frequency that is aligned with the first frequency.

Any feature of the electrosurgical apparatus and systems discussed herein may be utilised in the method.

Herein, the terms "proximal" and "distal" refer to the ends of a structure (e.g. electrosurgical instrument, coaxial feed cable, etc.) further from and closer to the treatment zone respectively. Thus, in use the proximal end of the structure is accessible by a user, whereas the distal end is closer to the treatment site, i.e. the patient.

The term "conductive" is used herein to mean electrically conductive, unless the context dictates otherwise.

The term "longitudinal" used below refers to the direction along the instrument channel parallel to the axis of the coaxial transmission line. The term "lateral" refers to a direction that is perpendicular to the longitudinal direction. The term "inner" means radially closer to the centre (e.g. axis) of the instrument channel. The term "outer" means radially further from the centre (axis) of the instrument channel.

BRIEF DESCRIPTION OF THE DRAWINGS

Examples of the invention are discussed below with reference to the accompanying drawings, in which:

Fig. 1 is a schematic diagram of an electrosurgical apparatus;
Fig. 2 is a schematic diagram of a part of an energy conveying device of the electrosurgical apparatus according to a first embodiment;
Fig. 3 is a schematic diagram of a part of an energy conveying device of the electrosurgical apparatus according to a second embodiment;
Fig. 4 is a schematic diagram of a part of an energy conveying device of the electrosurgical apparatus according to a third embodiment;
Fig. 5 is a schematic diagram of a part of an energy conveying device of the electrosurgical apparatus according to a fourth embodiment;
Fig. 6 is a schematic diagram depicting a set-up for measuring a leakage current; and
Fig. 7 is block diagram depicting steps of a method for reducing leakage current.

DETAILED DESCRIPTION; FURTHER OPTIONS AND PREFERENCES

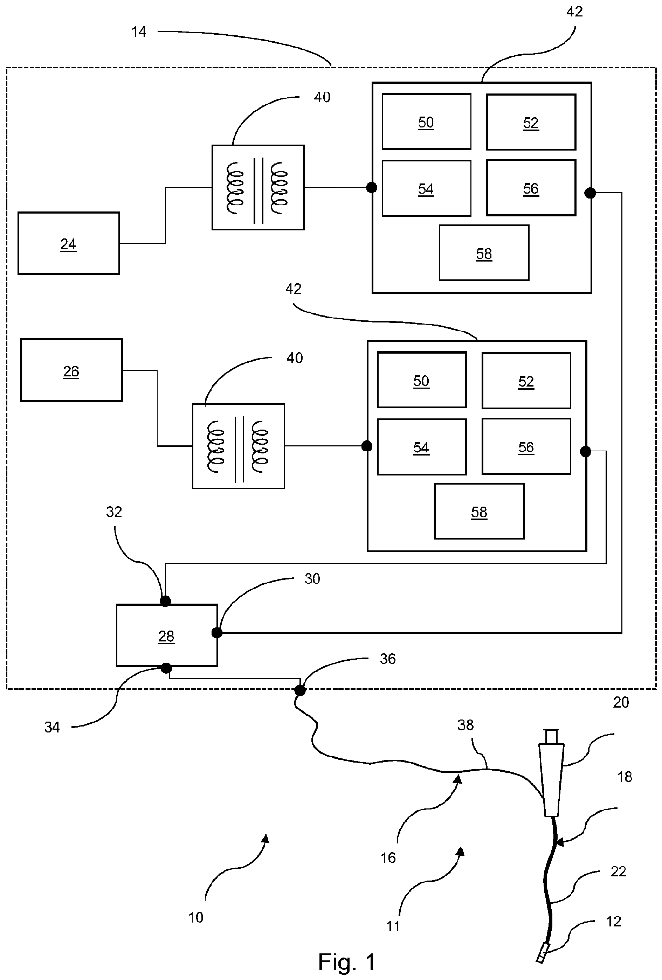

Fig. 1 is a schematic diagram of a complete electrosurgical apparatus 10 that is an embodiment of the invention. The apparatus 10 is arranged to treat biological tissue (e.g. a tumour, lesion or fibroid) using radiofrequency (RF) and/or microwave electromagnetic (EM) energy delivered from a radiating device 12. The EM energy emitted by the radiating device 12 into a treatment zone can be used to coagulate, cut, and/or ablate tissue in the treatment zone.

The electrosurgical apparatus 10 comprises a generator device 11 and the radiating device 12. The generator device 11 comprises a generator unit 14 for controllably supplying RF and/or microwave EM energy and an energy conveying device 16. The generator unit 14 may include one or more generators. A suitable generator for this purpose is described in WO 2012/076844, which is incorporated herein by reference. The generator unit 11 may be arranged to monitor reflected signals received back from the radiating device 12 in order to determine an appropriate power level for delivery. For example, the generator unit 14 may be arranged to calculate an impedance seen at the radiating device 12 in order to determine an optimal delivery power level.

The electrosurgical apparatus 10 further comprises a surgical scoping device 18, such as a bronchoscope, endoscope, gastroscope, laparoscope or the like. The scoping device 18 may include a handpiece 20 and a flexible shaft 22. The handpiece 20 may include means for guiding the flexible shaft 22 through a cavity of a body. For example, the handpiece 20 can include means for moving a distal end of the flexible shaft 22 to change direction of the distal end of the flexible shaft 22. This helps manoeuvring the flexible shaft 22 through the cavity of the body. The flexible shaft 22 may include a working channel through which elongated structures can be moved and, thus, positioned at the treatment zone within the cavity of the body.

The generator unit 14 can include a first generator 24 and a second generator 26 each of which is configured to generate electromagnetic radiation of a fixed frequency. However, the generator unit 14 is not limited thereto; the first generator 24 and/or the second generator 26 can be configured to generate AC electromagnetic energy in a continuous range between a minimum frequency and a maximum frequency. The frequency of the electromagnetic energy to be generated by the first generator 24 and/or the second generator 26 may be selected using an interface (not shown in the figures).

The generator unit 14 or the energy conveying device 16 can include a multiplexer 28 which includes a first input port 30, a second input port 32, and an output port 34. The first input port 30 is electrically coupled to the first generator 24 and a second input port 32 is electrically coupled to the second generator 26. The multiplexer 28 combines the inputs at the first input port 30 and the second input port 32 and outputs the combined inputs at the output port 34. Alternatively or additionally, the multiplexer 28 outputs the input at the first input port 30 or the input at the second input port 32 at the output port 34. In this embodiment, the first generator 24 and the second generator 26 operate in alternating manner, i.e. the first generator 24 or a second generator 26 are operating but not at the same time. However, it is also possible that the first generator 24 and the second generator 26 simultaneously operate. The output port 34 is electrically connected to a generator unit output 36 which is the output of the generator unit 14. In other words, electromagnetic energy generated by the generator unit 14 is output at the generator unit output 36 which is electrically connected to the energy conveying device 16. In the embodiment shown in Fig. 1, the multiplexer 28 is part of the generator unit 14. For example, the multiplexer 28 is arranged within a housing of the generator unit 14.

The energy conveying device 16 can include a transmission line 38, an isolation transformer 40, and a tuning control 42. In the embodiment shown in Fig. 1, the isolation transformer 40 and the tuning control 42 are part of the generator unit 14, for example, arranged within a housing of the generator unit 14. However, the invention is not limited thereto. The isolation transformer 40 and the tuning control 42 may be arranged outside of the generator unit 14. In other words, parts of the energy conveying device 16 can be arranged with a housing of the generator unit 14.

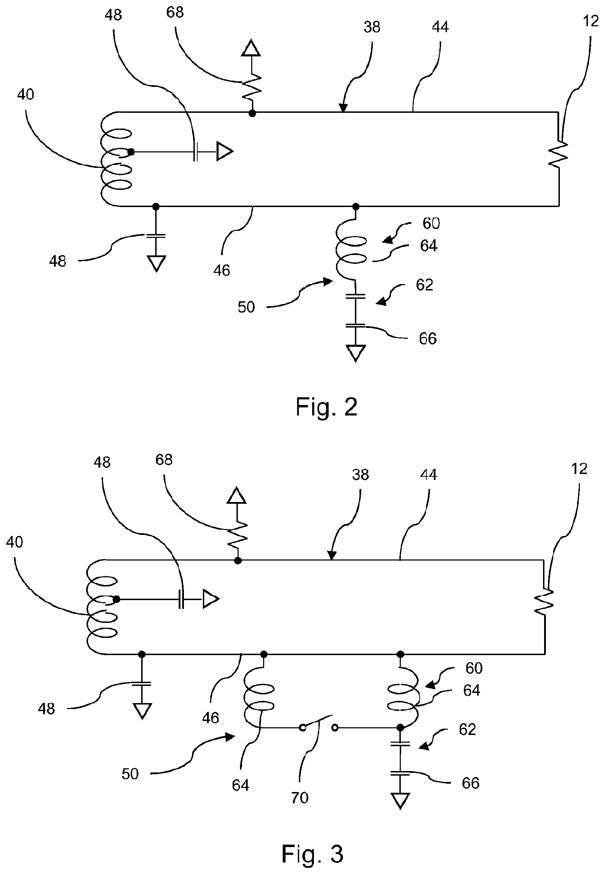

The transmission line 38 can include a first conductive line 44 (see Figs. 2 to 4), a second conductive line 46 (see Figs. 2 to 4), and an elongated body (not explicitly shown in the figures). The transmission line 38 is connected to the generator unit output 36 and a distal end of the transmission line 38 is connected to the radiating device 12 (not drawn to scale in Fig. 1). A part of the transmission line 38, of the first conductive line 44, and/or the second conductive line 46 can extend within the generator unit 14. The first conductive line 44 and the second conductive line 46 extend from the proximal end to the distal end and are covered by the elongated body.

The transmission line 38 can include a coaxial cable and is insertable (including the elongated body) through the entire length of the working channel of the flexible shaft 22. The radiating device 12 is shaped to pass through the working channel of the flexible shaft 22 and protrude (e.g. inside the patient) at the distal end of the working channel of the flexible shaft 22. The radiating device 12 includes a RF and/or microwave antenna for delivering RF and/or microwave energy into the treatment zone.

The structure of the radiating device 12 may be arranged to have a maximum outer diameter suitable for passing through the working channel. Typically, the diameter of a working channel in a surgical scoping device such as an endoscope is less than 4.0 mm, e.g. any one of 2.8 mm, 3.2 mm, 3.7 mm, 3.8mm. The length of the transmission line 38 can be equal to or greater than 1.2 m, e.g. 2 m or more. In other examples, the radiating device 12 may be mounted at the distal end of the transmission line 38 and the transmission line 38 has been inserted through the working channel (and before the flexible shaft 22 is introduced into the patient).

Alternatively, the transmission line 38 can be inserted into the working channel from the distal end before making its proximal connections. In these arrangements, the radiating device 12 can be permitted to have dimensions greater than the working channel of the surgical scoping device 114.

The scoping device 22 described above is one way of introducing the electrosurgical apparatus 10. Other techniques are possible. For example, the electrosurgical apparatus 10 may also be inserted using a catheter.

The isolation transformer 40 may be arranged within a housing of the generator unit 14. The insolation transformer 40 may be electrically connected between the first generator 24 or the second generator 26 and a respective tuning control 42 which in turn is electrically connected between the isolation transformer 40 and the multiplexer 28. The multiplexer 28 is electrically coupled to the first generator 24 and the second generator 26 via respective tuning controls 42 and isolation transformers 40.

A different isolation transformer 40 can be provided for each first generator 24 and the second generator 26. The different connection state of the multiplexer 28 may result in an increase or change in the leakage capacitance when all the individual leakage capacitances are added together. The isolation transformer 40 and/or the tuning control 42 may be omitted.

The isolation transformer 40 is an example of an electrical element which provides a first leakage capacitance. In other words, the transmission line 38 and the isolation transformer 40 are electrically isolated when a direct current (DC) is supplied to the isolation transformer 40 and the transmission line 38. However, in case of an alternating current (AC) supplied to the isolation transformer 40 and a transmission line 38 (for example, at a frequency for which the transmission line 38 is very short compared to a wavelength), the isolation transformer 40 has the electrical characteristics of a leakage capacitor 48 having the first leakage capacitance which is arranged between the transmission line 38 and the ground or neutral. Thus, during AC operation, a leakage current flows through the isolation transformer 40 (or the leakage capacitor 48) to the ground or neutral. The amount of the leakage current is dependent on the frequency of the electromagnetic energy supplied to the transmission line 38 and the isolation transformer 40. From an electrical point of view, the leakage capacitor 48 can be connected between the first conductive line 44 and the ground/neutral and/or between the second conductive line 46 and the ground/neutral (see also Figs. 2 to 4).

The isolation transformer 40 is provided for electrically isolating the first generator 24 and the second generator 26, respectively, from the radiating tip 12. The isolation transformer 40 may include two coils or windings which may act as or provide the characteristics of the leakage capacitor 48.

The tuning control 42 may include a tuning circuit 50, a measuring device 52, a display device 54, an input device 56, and/or a control device 58. The tuning control 42 will be described in more detail in conjunction with Figs. 2 to 4.

The transmission line 38 includes the first conductive line 44 and the second conductive line 46 which can be each electrically coupled or connected to the isolation transformer 40 (only one coil is depicted in Figs. 2 to 4) and the radiating device 12 which is represented by a resistor. The characteristics of the isolation transformer 40 as acting or providing a first leakage capacitance is schematically depicted by a leakage capacitor 48 electrically connected to the coil of the isolation transformer 40. However, the isolation transformer 40 may not be the only source of a leakage capacitance. For example, a microwave choke is provided to prevent leakage of microwave energy. Microwave isolation may also be provided by means of a separate microwave isolation transformer. The microwave choke or the separate microwave isolation transformer can act as a further or second leakage capacitance depicted in Figs. 2 to 5 as the leakage capacitor 48 connected to the second conductive line 46. This second leakage capacitor 48 may not be permanently connected to the second conductive line 46 such that only one of the two leakage capacitors 48 are connected to the second conductive line 46. This may require that the characteristics of the tuning circuit 60 need to be changed depending on which one of the leakage capacitors 48 is connected to the first conductive line 46. Further, the combination of the leakage capacitors 48 constitute the (first) leakage capacitance in case they are both connected to the second conductive line 46.

Please note that further sources for a leakage capacitance (providing a second leakage capacitance) can be present which basically depends on the configuration of the transmission line 38, the isolation transformer 40, the generator unit 14, and/or other electrical components.

To reduce or eliminate the leakage current, the tuning circuit 50 is provided. Due to the electrical connection of the tuning capacitors 48 in the embodiments of Figs. 2 to 4, the tuning circuit 50 is electrically connected between and second conductive line 46 and the ground/neutral. Thus, the tuning circuit 50 and the leakage capacitor 48 are connected in parallel between the second conductive line 46 and the ground/neutral.

The tuning circuit 50 includes an inductor unit 60 and a capacitor unit 62 which are connected in series between the second conductive line 46 and the ground/neutral. The inductor unit 60 includes one or more tuning inductors 64. In the embodiment of Fig. 2, the inductor unit 60 includes one tuning inductor 64. The capacitor unit 62 includes one or more capacitors 66. In the embodiment of Fig. 2, the capacitor unit 62 includes two capacitors 66. The capacitor unit 62 provides the DC isolation for the tuning circuit 50. The provision of only one capacitor 66 would be sufficient, however two capacitors 66 are provided for redundancy and, thus, for increased safety.

The tuning circuit 50 (in particular the inductor unit 60) and the leakage capacitor 48 form a parallel resonant circuit or LC circuit. In the embodiment of Fig. 2, a tuning inductance of the inductor unit 60 which is defined by the single tuning inductor 64 is selected such that the resonance frequency of the resonant circuit is equal to or close to the frequency that is generated by the generator unit 14 (the frequency of the electromagnetic energy propagating through the transmission line 38). In this case, the impedance of the resonant circuit is very high resulting in a significant reduction or a substantial prevention of the leakage current.

Please note that the reduction of the leakage current only works for frequencies of the electromagnetic radiation propagating within the transmission line 38 which are close to the resonance frequency of the resonant circuit. Thus, in the embodiment of Fig. 2, the generator unit 14 may only include the first generator 24 generating a fixed frequency that is close or equal to the resonance frequency of the resonant circuit. In this case, the measuring device 52, the display device 54, the input device 56, and/or the control device 58 may be omitted.

The capacitance of the capacitor unit 62 may be chosen to be an order of magnitude higher than the first leakage capacitance of the leakage capacitor 48. In this case, the capacitor unit 62 does not effectively interact with the electromagnetic energy propagating within the transmission line 38. In other words, the capacitor unit 62 does not substantially affect the resonance frequency of the tuning circuit 50 and/or of the resonant circuit. The capacitor unit 62 is provided for DC isolation.

The leakage current may be measured as the current flowing through a 200 $\Omega$ test resistor 68. The test resistor 68 may only be connected to transmission line 38 for the purpose of measuring or controlling the leakage current.

In one example, at 400 kHz, with 1 k$\Omega$ load of the radiating device 12, the total first leakage capacitance on one of the first conductive line 44 or the second conductive line 46, that would keep the power in the 200 $\Omega$ test resistor 68 on the opposite line below an acceptable limit, would be about 80 pF. The isolation transformer 40 and associated circuitry may have a capacitance of 60 pF. This contributes about 30 pF to each line of the first conductive line 44 or the second conductive line 46, so the permitted extra capacitance to ground is 50 pF. For operation at 400 kHz, capacitance of the capacitor unit 62 in the order of 1 $\mu$F in series with an inductance of the inductor unit 60 in the order of 1 mH would be suitable for cancelling a first leakage capacitance of the leakage capacitor 48 in the order of 100 pF.

In the embodiment depicted in Fig. 3, the tuning circuit 50 additionally includes a switch unit 70 to switch the tuning inductance of the inductor unit 60 between two inductances. The inductor unit 60 includes two tuning inductors 64, one of which is permanently connected in series with the capacitor unit 62 and the other tuning inductor 64 is only connected to the capacitor unit 62 if the switch unit 70 is in the closed state. In other words, in the open state of the switch unit 70, the tuning inductance of the inductor unit 60 is determined by the tuning inductor 64 which is permanently connected in series with the capacity unit 62. In the closed state of the switch unit 70, the tuning inductance of the inductor unit 60 is determined by both tuning inductors 64. Thus, the inductor unit 60 has two fixed tuning inductances which can be selected by operating the switch unit 70.

This provides two resonance frequencies of the resonant circuit such that leakage currents at two frequencies of the electromagnetic energy propagating within a transmission line 38 can be reduced. The embodiment of Fig. 3 may be used if the generator unit 14 includes the first generator 24 and the second generator 26 which each generate a fixed frequency. In this case, the inductances of the tuning inductors 64 are selected such that the respective resonance frequencies of the resonant circuit (in the open state and the closed state of the switch unit 70) are each close or equal to the frequency that is generated by the first generator 24 (e.g. first frequency) and a second generator 26 (e.g. second frequency), respectively.

Further, the switch unit 70 provides for the reduction or cancellation of leakage currents caused by different leakage capacitances (and thus resulting in different resonant frequencies of the resonant circuit). Thus, the switch unit 70 allows cancellation or reduction of leakage currents with different tools (such as radiating devices) and with different generators connected, which may have different leakage capacitances, which require different tuning inductances to cancel them at the same frequency.

It is also possible that the inductor unit 60 includes one or more tuning capacitors (not shown in Fig. 3) which may be used for finetuning the resonance frequency of the resonant circuit. Further, these one or more tuning capacitors may be switchable to the inductor unit 60 by the switch unit 70.

If the generator unit 14 is configured to generate three or more fixed frequencies(e.g. by including three or more generators), the tuning circuit 50 may include further switches or switch units 70 and the inductor unit 60 includes further tuning inductor 64 and/or tuning capacitors for providing three or more different resonance frequencies of the resonant circuit. This allows reducing or eliminating leakage currents at each of the frequencies generated by the generator unit 14 and/or caused by different leakage capacitances caused by different electrical components connected to the energy conveying device 16. In this embodiment, the measuring device 52, the display device 54, the input device 56, and/or the control device 58 may be omitted.

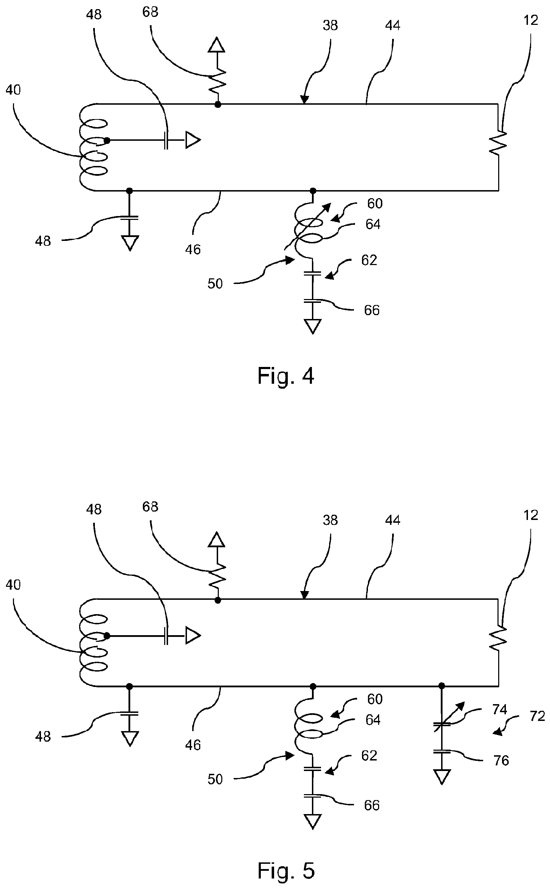

In the embodiment depicted in Fig. 4, the inductance of the inductor unit 60 is tuneable or changeable. This may be provided by a tuneable tuning inductor 64 whose inductance can be varied/changed. Alternatively or additionally, the resonance frequency of the resonant circuit may be tuneable by providing a tuneable capacitor (whose capacitance can be varied/changed) with the inductor unit 60. The inductance of the tuneable tuning inductor 64 may be tuned/changed/varied to such a value that the resonance frequency of the resonant circuit is equal to or close to the frequency that is generated by the generator unit 14.

This may be done manually. To this end, the measuring device 52 can be provided which is configured to measure the amount of the leakage current. Any known means for measuring a leakage current may be implemented by the measuring device 52. The measuring device 52 may be electrically and/or electronically connected to the display device 54 which can include a screen or display. The display device 54 can be configured to display the leakage current as measured by the measuring device 52. A user of the electrosurgical apparatus 10 can use the input device 56 for changing the inductance of the inductor unit 60 and/or for changing the resonance frequency of the resonant circuit (in case of tuneable tuning capacitor). The input device 56 can be a mechanical or electrical means for changing the inductance of the inductor unit 60 and/or the capacitance of the inductor unit 60. For example, the user operates the input device 56 in such a way that the leakage current is reduced.

The tuning of the inductor unit 60 may be automatically executed. To this end, the control device 58 can be provided which is connected to the measuring device 52 and/or the input device 56. The control device 58 may be a computer or the like which can receive the value of the leakage current from the measuring device 52 and controls the operation of the input device 56 for reducing the leakage current or directly controls the tuneable tuning inductor and/or the tuneable tuning capacitor.

It should be noted that different combinations of features from the embodiments shown in Figs. 2 to 4 are possible, with the embodiments shown in Figs. 2 to 4 being given merely by way of example. For example, the tuning circuit 50 may include both the switch unit 70 and a tuneable tuning inductor 64.

Further, a separate tuning circuit 50 can be used for each line of the first conductive line 44 and the second conductive line 46, if required. This separate tuning circuit 50 may have the same characteristics as the tuning circuit 50 depicted in Figs. 2 to 4.

In the embodiment depicted in Fig. 5, an additional capacitor unit 72 is provided between the ground and the second conductive line 46. The additional capacitor unit 72 is tuneable which means that an additional capacitance of the additional capacitor unit 72 is variable. A change in the additional capacitance changes the resonance frequency of the resonant circuit similar to the tuneable inductor unit 60 described in conjunction with Fig. 4. The difference is that the tuneable inductor unit 60 changes the inductance of the resonant circuit, whereas the additional capacitor unit 72 changes the capacitance of the resonant circuit. However, both changes result in a change of the resonance frequency.

The additional capacitor unit 72 may include a tuneable additional capacitor 74 and/or a redundancy additional capacitor 76. The tuneable additional capacitor 74 provides the tuneable characteristics of the additional capacitor unit 72. The redundancy additional capacitor 76 may be provided for redundancy reasons, i.e. if the tuneable additional capacitor 74, the redundancy additional capacitor 76 provides the DC isolation of the additional capacitor unit 72.

In a further embodiment not shown in the figures, the capacitor unit 62 may have the characteristics of the additional capacitor unit 72. Thus, in this embodiment, one of the capacitors 66 may be tuneable or variable. For example, one of the capacitors 66 may be replaced by the tuneable additional capacitor 74. Thus, the ground capacitance is variable so that the capacitor unit 62 may also contribute to the tuning of the tuning circuit 50.

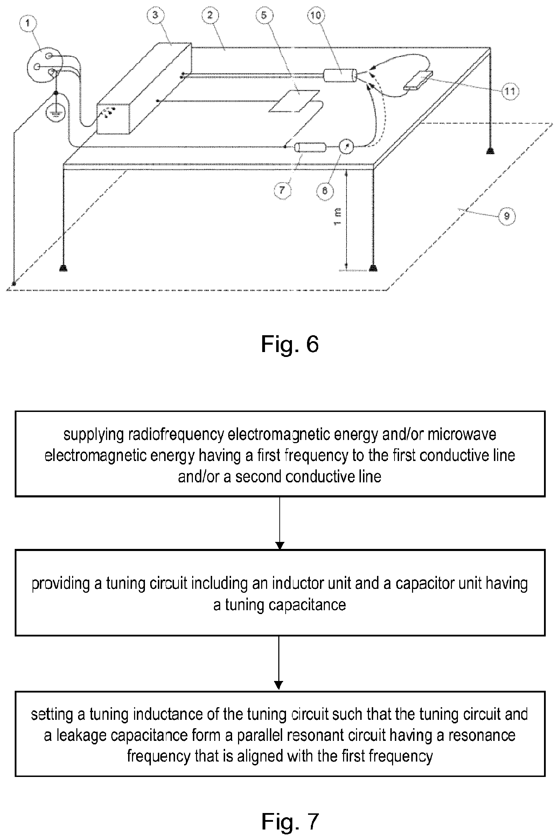

Fig. 6 depicts a set-up for measuring the leakage current. The electrosurgical apparatus 10 may be isolated from earth and from other applied parts at both high and low frequencies. The leakage current flowing from either pole of the bipolar output to earth and to the neutral electrode via a 200 Ω non-inductive resistor in each line should not exceed the value which produces a power in a 200 Ω non-inductive test resistor 68 equal to 1 % of the maximum bipolar rated output power, with all output controls set to maximum. This compliance can be checked by the following test.

The electrosurgical apparatus 10 is set up as shown in Fig. 6. The test is conducted using one side of the bipolar output and using bipolar and (if applicable) neutral electrode leads. The test is conducted with the output first being unloaded and then repeated with the output loaded at the rated load. The squared current value multiplied by 200 Ω test resistor 68 should not exceed the requirement above. The test is then repeated for the other side of the bipolar output.

The keys for Fig. 6 are: 1: supply mains, 2: table, made of isolating material; 3: electrosurgical apparatus 10; 5: neutral electrode, metallic or in contact with metal foil of the same size; 7: test resistance, 200 Ω; 8: measuring device 52 (high-frequency current meter); 9: earthed conductive plane; and 10: activated bipolar accessory (e.g. radiating device 12).

A method, not part of the invention, for reducing leakage current with the energy conveying device 16 is described in conjunction with Fig. 7. A first step is to supply radiofrequency and/or microwave electromagnetic energy to the first conductive line 44 and/or the second conductive line 46. This may be done by using the generator unit 14 as described above.

In a second step, the tuning circuit 50 as described in Figs. 2 to 5 is provided.

In third step, the tuning inductance of the tuning circuit 50 is set such that the tuning circuit 50 and the leakage capacitor 48 form a parallel resonant circuit having a resonance frequency that is equal to or close to the frequency that is generated by the generator unit 14. This setting step may be executed as described in conjunction with Figs. 2 to 5.

Please note that the first to third steps may not be executed in the order as described above. For example, the second step may be the step that is executed first.

**Claims**

1. An energy conveying device for conveying radiofrequency electromagnetic energy and/or microwave electromagnetic energy, comprising

   a first conductive line (44) and a second conductive line (46) configured to convey radiofrequency electromagnetic energy and/or microwave electromagnetic energy having a first frequency,
   an electrical element (40) providing a first leakage capacitance, the first leakage capacitance being between the first conductive line (44) and a ground and/or between the second conductive line (46) and the ground, and
   a tuning circuit (50) including an inductor unit (60) and a capacitor unit (62) having a ground capacitance, the inductor unit (60) and the capacitor unit (62) are connected in series between the ground and either the first conductive line (44) or the second conductive line (46),
   wherein the inductor unit (60) has a tuning inductance such that the tuning circuit (50) and the first leakage capacitance form a resonant circuit having a resonance frequency that is aligned with the first frequency, and
   **characterized in that**
   the energy conveying device (16) further includes an additional capacitor unit (72) having an additional capacitance that is variable, wherein the additional capacitor unit (72) is connected in series between the ground and either the first conductive line (44) or the second conductive line (46).

2. The energy conveying device of claim 1, wherein the ground capacitance of the capacitor unit (62) is greater than the first leakage capacitance, optionally by at least a factor of 10, 100, or 1000.

3. The energy conveying device of claim 1 or 2, wherein the inductor unit (60) includes one tuning inductor (64) having the tuning inductance or two or more tuning inductors (64) permanently connected to each other together providing the tuning inductance.

4. The energy conveying device of claim 1 or 2, wherein the inductor unit (60) includes

   two or more tuning inductors (64) and a switch unit (70), the switch unit (70) being configured to selectively electrically connect one or more tuning inductors (64) to the capacitor unit (62) for selectively changing the tuning inductance of the tuning circuit (50), or
   at least one tuning inductor (64), at least one tuning capacitor, and a switch unit (70), the switch unit (70) being configured to selectively electrically connect one or more tuning inductors (64) and/or one or more tuning capacitor to the capacitor unit (62) for selectively changing the resonance frequency of the resonant circuit.

5. The energy conveying device of claim 4, wherein

the first conductive line (44) and the second conductive line (46) are configured to convey radiofrequency electromagnetic energy and/or microwave electromagnetic energy having a second frequency which is different to the first frequency, and/or
the electrical element (40) provides a second leakage capacitance which is different to the first leakage capacitance,
wherein
the tuning circuit (50) is configured to selectively change the resonance frequency of the resonant circuit such that the resonance frequency is aligned with the first frequency or the second frequency and/or
the tuning circuit (50) is configured to selectively change the resonant circuit such that the tuning circuit (50) and the first or second leakage capacitances form a resonant circuit having respective resonance frequencies that are aligned with the first frequency.

6. The energy conveying device of claim 1 or 2, wherein the inductor unit (60) includes at least one tuning inductor (64) and/or at least one tuning capacitor that are variable for changing the resonance frequency of the resonant circuit, wherein optionally

the first conductive line (44) and the second conductive line (46) are configured to convey the radiofrequency electromagnetic energy and/or microwave electromagnetic energy having a second frequency which is different to the first frequency, and/or
the electrical element (40) provides a second leakage capacitance which is different to the first leakage capacitance,
wherein
the tuning circuit (50) is configured to be varied to selectively change the resonance frequency of the resonant circuit such that the resonance frequency is aligned with the first frequency or the second frequency and/or
the tuning circuit (50) is configured to selectively change the tuning circuit (50) such that the tuning circuit (50) and the first or second leakage capacitances form a resonant circuit having respective resonance frequencies that are aligned with the first frequency.

7. The energy conveying device of any preceding claim, wherein

the first conductive line (44) and the second conductive line (46) are configured to convey the radiofrequency electromagnetic energy and/or microwave electromagnetic energy having a second frequency which is different to the first frequency, and/or
the electrical element (40) provides a second leakage capacitance which is different to the first leakage capacitance,
wherein
the additional capacitor unit (72) is configured to be varied to selectively change the resonance frequency of the resonant circuit such that the resonance frequency is aligned with the first frequency or the second frequency and/or
the additional capacitor unit (72) is configured to selectively change the tuning circuit (50) such that the tuning circuit (50) and the first or second leakage capacitances form a resonant circuit having respective resonance frequencies that are aligned with the first frequency.

8. The energy conveying device of any preceding claim, further comprising a measuring device (52) for measuring and/or monitoring a leakage current induced by the first leakage capacitance,

wherein optionally the energy conveying device (16) further comprises
a display device (54) for displaying the leakage current measured by the measuring device (52), and
an input device (56) for changing the resonance frequency of the resonant circuit by varying the at least one tuning inductor (64) and/or the at least one tuning capacitor.

9. The energy conveying device of claim 8, further comprising a control device (58) configured to vary the at least one tuning inductor (64), the additional capacitor unit (72), and/or the at least one tuning capacitor to change the resonance frequency of the resonant circuit based on the leakage current measured by the measuring device (52).

10. The energy conveying device of any preceding claim,

     wherein the capacitor unit (62) comprises two or more capacitors (66), optionally connected together in series, and/or
     wherein the electrical element (40) includes an isolation transformer, a microwave choke, and/or a multiplexer (28).

11. The energy conveying device of any preceding claim, further comprising a second tuning circuit (50) including a second inductor unit (60) and a second capacitor unit (62) having a second ground capacitance, the inductor unit (60) and the capacitor unit (62) being connected in series between the ground and either the first conductive line (44) or the second conductive line (46), the second inductor unit (60) and the second capacitor unit (62) being connected in series between the ground and the other one of the first conductive line (44) and the second conductive line (46),
     wherein the second inductor unit (60) has a second tuning inductance such that the second tuning circuit (50) and the first leakage capacitance form a resonant circuit having a second resonance frequency that is aligned with the first frequency.

12. A generator device for generating and/or conveying radiofrequency electromagnetic energy and/or microwave electromagnetic energy, comprising

     a generator unit (14) configured to generate radiofrequency electromagnetic energy and/or microwave electromagnetic energy having the first frequency, and
     the energy conveying device (16) of any preceding claim, the energy conveying device (16) being electrically coupled to the generator unit (14) so as to receive the radiofrequency electromagnetic energy and/or microwave electromagnetic energy having the first frequency.

13. The generator device of claim 12, wherein the generator unit (14) comprises

     a first generator (24) configured to generate radiofrequency electromagnetic energy and/or microwave electromagnetic energy having the first frequency,
     a second generator (26) configured to generate radiofrequency electromagnetic energy and/or microwave electromagnetic energy having a second frequency, the second frequency being different to the first frequency, and
     a multiplexer (28) including a first input port (30), a second input port (32), and an output port (34),
     wherein the first generator (24) is coupled to the first input port (30), the second generator (26) is coupled to the second input port (32), and the energy conveying device (16) is coupled to the output port (34).

14. An electrosurgical apparatus, comprising

     the generator device of claims 12 or 13, and
     a radiating device (12) for emitting radiofrequency electromagnetic energy and/or microwave electromagnetic energy.

15. The electrosurgical apparatus of claim 14, further comprising an elongate body configured to be inserted in a working channel of a surgical scoping device (18) and including a proximal end and a distal end,

     wherein radiating device (12) is attached to the distal end, and
     wherein the first conductive line (44) and the second conductive line (46) extend in the elongate body.

**Patentansprüche**

1. Energieübertragungsvorrichtung zur Übertragung von elektromagnetischer Hochfrequenz-Energie und/oder elektromagnetischer Mikrowellenenergie, umfassend:

     eine erste leitfähige Leitung (44) und eine zweite leitfähige Leitung (46), die dazu ausgelegt sind, elektromagnetische Hochfrequenzenergie und/oder elektromagnetische Mikrowellenenergie mit einer ersten Frequenz zu übertragen,
     ein elektrisches Element (40), das eine erste Leckkapazität bereitstellt, wobei die erste Leckkapazität zwischen der ersten leitfähigen Leitung (44) und einer Masse und/oder zwischen der zweiten leitfähigen Leitung (46) und der Masse ist, und

eine Abstimmschaltung (50), die eine Induktoreinheit (60) und eine Kondensatoreinheit (62) mit einer Massekapazität aufweist, wobei die Induktoreinheit (60) und die Kondensatoreinheit (62) zwischen die Masse und entweder die erste leitfähige Leitung (44) oder die zweite leitfähige Leitung (46) in Serie geschaltet sind, wobei die Induktoreinheit (60) eine Abstimminduktivität aufweist, sodass die Abstimmschaltung (50) und die erste Leckkapazität eine Resonanzschaltung ausbilden, die eine Resonanzfrequenz aufweist, welche auf die erste Frequenz abgestimmt ist, und

**dadurch gekennzeichnet, dass** die Energieübertragungsvorrichtung (16) ferner eine zusätzliche Kondensatoreinheit (72) mit einer zusätzlichen Kapazität, die variabel ist, umfasst, wobei die zusätzliche Kondensatoreinheit (72) zwischen die Masse und entweder die erste leitfähige Leitung (44) oder die zweite leitfähige Leitung (46) in Serie geschaltet ist.

2. Energieübertragungsvorrichtung nach Anspruch 1, wobei die Massekapazität der Kondensatoreinheit (62) größer als die erste Leckkapazität ist, gegebenenfalls um zumindest einen Faktor von 10, 100 oder 1000.

3. Energieübertragungsvorrichtung nach Anspruch 1 oder 2, wobei die Induktoreinheit (60) einen Abstimminduktor (64), der die Abstimminduktivität aufweist, oder zwei oder mehrere Abstimminduktoren (64), die permanent miteinander verbunden sind, die zusammen die Abstimminduktivität bereitstellen, umfasst.

4. Energieübertragungsvorrichtung nach Anspruch 1 oder 2, wobei die Induktoreinheit (60) ferner Folgendes umfasst:

zwei oder mehrere Abstimminduktoren (64) und eine Schalteinheit (70), wobei die Schalteinheit (70) dazu ausgelegt ist, selektiv einen oder mehrere Abstimminduktoren (64) elektrisch mit der Kondensatoreinheit (62) zu verbinden, um selektiv die Abstimminduktivität der Abstimmschaltung (50) zu ändern, oder zumindest einen Abstimminduktor (64), zumindest einen Abstimmkondensator und eine Schalteinheit (70), wobei die Schalteinheit (70) dazu ausgelegt ist, selektiv einen oder mehrere Abstimminduktoren (64) und/oder einen oder mehrere Abstimmkondensatoren elektrisch mit der Kondensatoreinheit (62) zu verbinden, um selektiv die Resonanzfrequenz der Resonanzschaltung zu ändern.

5. Energieübertragungsvorrichtung nach Anspruch 4, wobei

die erste leitfähige Leitung (44) und die zweite leitfähige Leitung (46) dazu ausgelegt sind, elektromagnetische Hochfrequenzenergie und/oder elektromagnetische Mikrowellenenergie mit einer zweiten Frequenz, die sich von der ersten Frequenz unterscheidet, zu übertragen, und/oder das elektrische Element (40) eine zweite Leckkapazität bereitstellt, die sich von der ersten Leckkapazität unterscheidet, wobei

die Abstimmschaltung (50) dazu ausgelegt ist, die Resonanzfrequenz der Resonanzschaltung selektiv derart zu ändern, dass die Resonanzfrequenz auf die erste Frequenz oder die zweite Frequenz abgestimmt ist, und/oder die Abstimmschaltung (50) dazu ausgelegt ist, selektiv die Resonanzschaltung derart zu ändern, dass die Abstimmschaltung (50) und die erste oder zweite Leckkapazität eine Resonanzschaltung ausbilden, die entsprechende Resonanzfrequenzen aufweist, welche auf die erste Frequenz abgestimmt sind.

6. Energieübertragungsvorrichtung nach Anspruch 1 oder 2, wobei die Induktoreinheit (60) zumindest einen Abstimminduktor (64) und/oder zumindest einen Abstimmkondensator aufweist, die variabel sind, um die Resonanzfrequenz der Resonanzschaltung zu ändern,

wobei gegebenenfalls
die erste leitfähige Leitung (44) und die zweite leitfähige Leitung (46) dazu ausgelegt sind, die elektromagnetische Hochfrequenzenergie und/oder elektromagnetische Mikrowellenenergie mit einer zweiten Frequenz, die sich von der ersten Frequenz unterscheidet, zu übertragen, und/oder das elektrische Element (40) eine zweite Leckkapazität bereitstellt, die sich von der ersten Leckkapazität unterscheidet, wobei

die Abstimmschaltung (50) dazu ausgelegt ist, variiert zu werden, um selektiv die Resonanzfrequenz der Resonanzschaltung derart zu ändern, dass die Resonanzfrequenz auf die erste Frequenz oder die zweite Frequenz abgestimmt ist, und/oder

die Abstimmschaltung (50) dazu ausgelegt ist, selektiv die Abstimmschaltung (50) derart zu ändern, dass die Abstimmschaltung (50) und die erste oder zweite Leckkapazität eine Resonanzschaltung ausbilden, die entsprechende Resonanzfrequenzen aufweist, welche auf die erste Frequenz abgestimmt sind.

7. Energieübertragungsvorrichtung nach einem der vorangegangenen Ansprüche, wobei

die erste leitfähige Leitung (44) und die zweite leitfähige Leitung (46) dazu ausgelegt sind, die elektromagnetische Hochfrequenzenergie und/oder elektromagnetische Mikrowellenenergie mit einer zweiten Frequenz, die sich von der ersten Frequenz unterscheidet, zu übertragen, und/oder
das elektrische Element (40) eine zweite Leckkapazität bereitstellt, die sich von der ersten Leckkapazität unterscheidet,
wobei
die zusätzliche Kondensatoreinheit (72) dazu ausgelegt ist, variiert zu werden, um selektiv die Resonanzfrequenz der Resonanzschaltung derart zu ändern, dass die Resonanzfrequenz auf die erste Frequenz oder die zweite Frequenz abgestimmt ist, und/oder
die zusätzliche Kondensatoreinheit (72) dazu ausgelegt ist, selektiv die Abstimmschaltung (50) derart zu ändern, dass die Abstimmschaltung (50) und die erste oder zweite Leckkapazität eine Resonanzschaltung ausbilden, die entsprechende Resonanzfrequenzen aufweist, welche auf die erste Frequenz abgestimmt sind.

8. Energieübertragungsvorrichtung nach einem der vorangegangenen Ansprüche, ferner umfassend eine Messvorrichtung (52) zum Messen und/oder Überwachen eines Leckstroms, der von der ersten Leckkapazität hervorgerufen wird,
wobei die Energieübertragungsvorrichtung (16) gegebenenfalls ferner Folgendes umfasst:

eine Anzeigevorrichtung (54) zum Anzeigen des von der Messvorrichtung (52) gemessenen Leckstroms, und
eine Eingabevorrichtung (56) zum Ändern der Resonanzfrequenz der Resonanzschaltung durch Variieren des zumindest einen Abstimminduktors (64) und/oder des zumindest einen Abstimmkondensators.

9. Energieübertragungsvorrichtung nach Anspruch 8, ferner umfassend eine Steuervorrichtung (58), die dazu ausgelegt ist, den zumindest einen Abstimminduktor (64), die zusätzliche Kondensatoreinheit (72) und/oder den zumindest einen Abstimmkondensator zu variieren, um die Resonanzfrequenz der Resonanzschaltung beruhend auf dem durch die Messvorrichtung (52) gemessenen Leckstrom zu ändern.

10. Energieübertragungsvorrichtung nach einem der vorangegangenen Ansprüche,

wobei die Kondensatoreinheit (62) zwei oder mehrere Kondensatoren (66), die gegebenenfalls miteinander in Serie geschaltet sind, umfasst, und/oder
wobei das elektrische Element (40) einen Trenntransformator, eine Mikrowellendrossel und/oder einen Multiplexer (28) umfasst.

11. Energieübertragungsvorrichtung nach einem der vorangegangenen Ansprüche, ferner umfassend eine zweite Abstimmschaltung (50), die eine zweite Induktoreinheit (60) und eine zweite Kondensatoreinheit (62) mit einer zweiten Massekapazität umfasst, wobei die Induktoreinheit (60) und die Kondensatoreinheit (62) zwischen die Masse und entweder die erste leitfähige Leitung (44) oder die zweite leitfähige Leitung (46) in Serie geschaltet sind, wobei die zweite Induktoreinheit (60) und die zweite Kondensatoreinheit (62) zwischen die Masse und die andere aus der ersten leitfähigen Leitung (44) und der zweiten leitfähigen Leitung (46) in Serie geschaltet sind,
wobei die zweite Induktoreinheit (60) eine zweite Abstimminduktivität aufweist, sodass die zweite Abstimmschaltung (50) und die erste Leckkapazität eine Resonanzschaltung ausbilden, die eine zweite Resonanzfrequenz aufweist, welche auf die erste Frequenz abgestimmt ist.

12. Generatorvorrichtung zum Erzeugen und/oder Übertragen von elektromagnetischer Hochfrequenzenergie und/oder elektromagnetischer Mikrowellenenergie, umfassend:

eine Generatoreinheit (14), die dazu ausgelegt ist, elektromagnetische Hochfrequenzenergie und/oder elektromagnetische Mikrowellenenergie mit der ersten Frequenz zu erzeugen, und
eine Energieübertragungseinheit (16) nach einem der vorangegangenen Ansprüche, wobei die Energieübertragungsvorrichtung (16) elektrisch mit der Generatoreinheit (14) gekoppelt ist, um die elektromagnetische Hochfrequenzenergie und/oder die elektromagnetische Mikrowellenenergie mit der ersten Frequenz zu empfangen.

13. Generatorvorrichtung nach Anspruch 12, wobei die Generatoreinheit (14) Folgendes umfasst:

einen ersten Generator (24), der dazu ausgelegt ist, elektromagnetische Hochfrequenzenergie und/oder elektromagnetische Mikrowellenenergie mit der ersten Frequenz zu erzeugen,

einen zweiten Generator (26), der dazu ausgelegt ist, elektromagnetische Hochfrequenzenergie und/oder elektromagnetische Mikrowellenenergie mit einer zweiten Frequenz zu erzeugen, wobei die zweite Frequenz sich von der ersten Frequenz unterscheidet, und

einen Multiplexer (28), der einen ersten Eingangsanschluss (30), einen zweiten Eingangsanschluss (32) und einen Ausgangsanschluss (34) umfasst,

wobei der erste Generator (24) mit dem ersten Eingangsanschluss (30) gekoppelt ist, der zweite Generator (26) mit dem zweiten Eingangsanschluss (32) gekoppelt ist und die Energieübertragungsvorrichtung (16) mit dem Ausgangsanschluss (34) gekoppelt ist.

14. Elektrochirurgische Vorrichtung, umfassend

eine Generatorvorrichtung nach Anspruch 12 oder 13 und

eine Strahlungsvorrichtung (12) zum Emittieren von elektromagnetischer Hochfrequenzenergie und/oder elektromagnetischer Mikrowellenenergie.

15. Elektrochirurgische Vorrichtung nach Anspruch 14, ferner umfassend einen länglichen Körper, der dazu ausgelegt ist, in einen Arbeitskanal einer chirurgischen Sondierungsvorrichtung (18) eingebracht zu sein und ein proximales Ende und ein distales Ende umfasst,

wobei die Strahlungsvorrichtung (12) an dem distalen Ende befestigt ist und

wobei die erste leitfähige Leitung (44) und die zweite leitfähige Leitung (46) sich im länglichen Körper erstrecken.

## Revendications

1. Dispositif de transport d'énergie destiné à transporter une énergie électromagnétique radiofréquence et/ou une énergie électromagnétique à micro-ondes, comprenant

une première ligne conductrice (44) et une deuxième ligne conductrice (46) configurées pour transporter une énergie électromagnétique radiofréquence et/ou une énergie électromagnétique à micro-ondes ayant un première fréquence,

un élément électrique (40) qui fournit une première capacitance de fuite, la première capacitance de fuite se trouvant entre la première ligne conductrice (44) et une masse et/ou entre la deuxième ligne conductrice (46) et la masse, et

un circuit de réglage (50) comportant une unité d'inducteur (60) et une unité de condensateur (62) ayant une capacitance de masse, l'unité d'inducteur (60) et l'unité de condensateur (62) étant reliées en série entre la masse et soit la première ligne conductrice (44), soit la deuxième ligne conductrice (46),

dans lequel l'unité d'inducteur (60) présente une inductance de réglage telle que le circuit de réglage (50) et la première capacitance de fuite forment un circuit résonnant ayant une fréquence de résonance qui est alignée avec la première fréquence, et

**caractérisé en ce que**

le dispositif de transport d'énergie (16) comporte en outre une unité de condensateur supplémentaire (72) ayant une capacitance supplémentaire qui est variable, dans lequel l'unité de condensateur supplémentaire (72) est reliée en série entre la masse et soit la première ligne conductrice (44), soit la deuxième ligne conductrice (46).

2. Dispositif de transport d'énergie selon la revendication 1, dans lequel la capacitance de masse de l'unité de condensateur (62) est supérieure à la première capacitance de fuite, éventuellement selon au moins un facteur de 10, 100 ou 1000.

3. Dispositif de transport d'énergie selon la revendication 1 ou 2, dans lequel l'unité d'inducteur (60) comporte un inducteur de réglage (64) ayant l'inductance de réglage de deux ou plus inducteurs de réglage (64) reliés en permanence l'un à l'autre et fournissant l'inductance de réglage.

4. Dispositif de transport d'énergie selon la revendication 1 ou 2, dans lequel l'unité d'inducteur (60) comporte

deux ou plus unités d'inducteurs de réglage (64) et une unité de commutateur (70), l'unité de commutateur (70) étant configurée pour relier électriquement de manière sélective un ou plusieurs inducteur(s) de réglage (64) à l'unité de condensateur (62) afin de changer sélectivement l'inductance de réglage du circuit de réglage (50), ou au moins un inducteur de réglage (64), au moins un condensateur de réglage, et une unité de commutateur (70), l'unité de commutateur (70) étant configurée pour relier électriquement de manière sélective un ou plusieurs inducteur(s) de réglage (64) et/ou un ou plusieurs condensateur (s) de réglage à l'unité de condensateur (62) afin de changer sélectivement la fréquence de résonance du circuit résonnant.

5. Dispositif de transport d'énergie selon la revendication 4, dans lequel

la première ligne conductrice (44) et la deuxième ligne conductrice (46) sont configurées pour transporter une énergie électromagnétique radiofréquence et/ou une énergie électromagnétique à micro-ondes ayant une deuxième fréquence qui est différente de la première fréquence, et/ou
l'élément électrique (40) fournit une deuxième capacitance de fuite qui est différente de la première capacitance de fuite,
dans lequel
le circuit de réglage (50) est configuré pour changer sélectivement la fréquence de résonance du circuit résonnant de sorte que la fréquence de résonance soit alignée avec la première fréquence ou la deuxième fréquence, et/ou
le circuit de réglage (50) est configuré pour changer sélectivement le circuit résonnant de sorte que le circuit de réglage (50) et la première ou la deuxième capacitance de fuite forment un circuit résonnant ayant des fréquences de résonance respectives qui soient alignées avec la première fréquence.

6. Dispositif de transport d'énergie selon la revendication 1 ou 2, dans lequel l'unité d'inducteur (60) comporte au moins un inducteur de réglage (64) et/ou au moins un condensateur de réglage qui sont variables afin de changer la fréquence de résonance du circuit résonnant,

dans lequel, éventuellement,
la première ligne conductrice (44) et la deuxième ligne conductrice (46) sont configurées pour transporter l'énergie électromagnétique radiofréquence et/ou l'énergie électromagnétique à micro-ondes ayant une deuxième fréquence qui est différente de la première fréquence, et/ou
l'élément électrique (40) fournit une deuxième capacitance de fuite qui est différente de la première capacitance de fuite,
dans lequel
le circuit de réglage (50) est configuré pour être varié afin de changer sélectivement la fréquence de résonance du circuit résonnant de sorte que la fréquence de résonance soit alignée avec la première fréquence ou la deuxième fréquence, et/ou
le circuit de réglage (50) est configuré pour changer sélectivement le circuit de réglage (50) de sorte que le circuit de réglage (50) et la première ou la deuxième capacitance de réglage forment un circuit résonnant ayant des fréquences de résonance respectives qui soient alignées avec la première fréquence.

7. Dispositif de transport d'énergie selon l'une quelconque des revendications précédentes, dans lequel

la première ligne conductrice (44) et la deuxième ligne conductrice (46) sont configurées pour transporter l'énergie électromagnétique radiofréquence et/ou l'énergie électromagnétique à micro-ondes ayant une deuxième fréquence qui est différente de la première fréquence, et/ou
l'élément électrique (40) fournit une deuxième capacitance de fuite qui est différente de la première capacitance de fuite,
dans lequel
l'unité de condensateur supplémentaire (72) est configurée pour être variée afin de changer sélectivement la fréquence de résonance du circuit résonnant de sorte que la fréquence de résonance soit alignée avec la première fréquence ou la deuxième fréquence, et/ou
l'unité de condensateur supplémentaire (72) est configurée pour changer sélectivement le circuit de réglage (50) de sorte que le circuit de réglage (50) et la première ou la deuxième capacitance de fuite forment un circuit résonnant ayant des fréquences de résonance respectives qui soient alignées avec la première fréquence.

8. Dispositif de transport d'énergie selon l'une quelconque des revendications précédentes, comprenant en outre un dispositif de mesure (52) destiné à mesurer et/ou surveiller un courant de fuite induit par la première capacitance de fuite,

dans lequel, éventuellement, le dispositif de transport d'énergie (16) comprend en outre
un dispositif d'affichage (54) destiné à afficher le courant de fuite mesuré par le dispositif de mesure (52), et
un dispositif d'entrée (56) destiné à changer la fréquence de résonance du circuit résonnant en faisant varier l'au moins un inducteur de réglage (64) et/ou l'au moins un condensateur de réglage.

9.  Dispositif de transport d'énergie selon la revendication 8, comprenant en outre un dispositif de commande (58) configuré pour faire varier l'au moins un inducteur de réglage (64), l'unité de condensateur supplémentaire (72), et/ou l'au moins un condensateur de réglage afin de changer la fréquence de résonance du circuit résonnant sur la base du courant de fuite mesuré par le dispositif de mesure (52).

10. Dispositif de transport d'énergie selon l'une quelconque des revendications précédentes,

dans lequel l'unité de condensateur (62) comprend deux ou plus condensateurs (66), éventuellement reliés ensemble en série, et/ou
dans lequel l'élément électrique (40) comporte un transformateur d'isolation, une bobine d'arrêt de micro-ondes, et/ou un multiplexeur (28).

11. Dispositif de transport d'énergie selon l'une quelconque des revendications précédentes, comprenant en outre un deuxième circuit de réglage (50) comportant une deuxième unité d'inducteur (60) et une deuxième unité de condensateur (62) ayant une deuxième capacitance de masse, l'unité d'inducteur (60) et l'unité de condensateur (62) étant reliées en série entre la masse et soit la première ligne conductrice (44), soit la deuxième ligne conductrice (46), la deuxième unité d'inducteur (60) et la deuxième unité de condensateur (62) étant reliées en série entre la masse et l'autre de la première ligne conductrice (44) et de la deuxième ligne conductrice (46),
dans lequel la deuxième unité d'inducteur (60) présente une deuxième inductance de réglage de sorte que le circuit de réglage (50) et la première capacitance de fuite forment un circuit résonnant ayant une deuxième fréquence de résonance qui soit alignée avec la première fréquence.

12. Dispositif générateur destiné à générer et/ou transporter une énergie électromagnétique radiofréquence et/ou une énergie électromagnétique à micro-ondes, comprenant

une unité de générateur (14) configurée pour générer une énergie électromagnétique radiofréquence et/ou une énergie électromagnétique à micro-ondes ayant la première fréquence, et
le dispositif de transport d'énergie (16) selon l'une quelconque des revendications précédentes, le dispositif de transport d'énergie (16) étant couplé électriquement à l'unité de générateur (14) de façon à recevoir l'énergie électromagnétique radiofréquence et/ou l'énergie électromagnétique à micro-ondes ayant la première fréquence.

13. Dispositif générateur selon la revendication 12, dans lequel l'unité de générateur (14) comprend

un premier générateur (24) configuré pour générer une énergie électromagnétique radiofréquence et/ou une énergie électromagnétique à micro-ondes ayant la première fréquence,
un deuxième générateur (26) configuré pour générer une énergie électromagnétique radiofréquence et/ou une énergie électromagnétique à micro-ondes ayant une deuxième fréquence, la deuxième fréquence étant différente de la première fréquence, et
un multiplexeur (28) comportant un premier port d'entrée (30), un deuxième port d'entrée (32), et un port de sortie (34),
dans lequel le premier générateur (24) est couplé au premier port d'entrée (30), le deuxième générateur (26) est couplé au deuxième port d'entrée (32), et le dispositif de transport d'énergie (16) est couplé au port de sortie (34).

14. Appareil électrochirurgical, comprenant

le dispositif générateur selon les revendications 12 ou 13, et
un dispositif rayonnant (12) destiné à émettre une énergie électromagnétique radiofréquence et/ou une énergie électromagnétique à micro-ondes.

15. Appareil électrochirurgical selon la revendication 14, comprenant en outre un corps allongé configuré pour être inséré dans un canal de fonctionnement d'un dispositif d'exploration chirurgicale (18) et comportant une extrémité proximale et une extrémité distale,

dans lequel le dispositif rayonnant (12) est fixé sur l'extrémité distale, et
dans lequel la première ligne conductrice (44) et la deuxième ligne conductrice (46) s'étendent dans le corps allongé.

Fig. 1

Fig. 2

Fig. 3

Fig. 4

Fig. 5

Fig. 6

supplying radiofrequency electromagnetic energy and/or microwave electromagnetic energy having a first frequency to the first conductive line and/or a second conductive line

providing a tuning circuit including an inductor unit and a capacitor unit having a tuning capacitance

setting a tuning inductance of the tuning circuit such that the tuning circuit and a leakage capacitance form a parallel resonant circuit having a resonance frequency that is aligned with the first frequency

Fig. 7

**EP 4 486 238 B1**

## REFERENCES CITED IN THE DESCRIPTION

**Patent documents cited in the description**

- US 3946738 A
- EP 1854423 A2
- US 2019274717 A1
- WO 2012076844 A